# EUROPÄISCHE PATENTANMELDUNG

(11) **EP 3 530 300 A1**
(43) Veröffentlichungstag der Anmeldung: **28.08.2019**
(21) Anmeldenummer: 19159385.4
(22) Anmeldetag: 26.02.2019
(51) Int. Cl.: A61M 1/14, A61M 1/16, A61M 39/10

(54) **ANSCHLUSSEINHEIT FÜR EINE BLUTBEHANDLUNGSMASCHINE ZUM ANSCHLIESSEN DER BLUTBEHANDLUNGSMASCHINE AN EINEN EXTERNEN BEHÄLTER UND BLUTBEHANDLUNGSMASCHINE MIT EINER DERARTIGEN ANSCHLUSSEINHEIT**

(30) Priorität: 27.02.2018 DE 102018104457
(71) Anmelder: B. Braun Avitum AG, 34212 Melsungen (DE)
(72) Erfinder: JANIK, Waldemar, 34121 Melsungen (DE); DURU, Jens, 36179 Bebra (DE)
(74) Vertreter: Winter, Brandl, Fürniss, Hübner, Röss, Kaiser, Polte - Partnerschaft mbB

(57) **Zusammenfassung**

Die vorliegende Erfindung betrifft eine Anschlusseinheit für eine Blutbehandlungsmaschine zum Anschließen der Blutbehandlungsmaschine an einen externen Behälter mit einer maschinenseitigen Leitung welche dazu ausgelegt ist mit der Blutbehandlungsmaschine fluidisch verbunden zu werden, einer behälterseitigen Leitung, welche dazu ausgelegt ist mit dem Behälter fluidisch verbunden zu werden und einem fest mit der maschinenseitigen Leitung und der behälterseitigen Leitung verbundenen Aufsatz, welcher dazu ausgelegt ist an einem Anschlussstutzen des externen Behälters lösbar montiert zu werden, um den externen Behälter fluidisch mit der Blutbehandlungsmaschine zu verbinden; und eine Blutbehandlungsmaschine mit einer derartigen Anschlusseinheit.

## Beschreibung

Die vorliegende Erfindung betrifft eine Anschlusseinheit für eine Blutbehandlungsmaschine zum Anschließen der Blutbehandlungsmaschine an einen externen Behälter, ein System aus einer derartigen Anschlusseinheit und einem externen Behälter, sowie eine Blutbehandlungsmaschine mit mindestens einer derartigen Anschlusseinheit.

### Hintergrund der Erfindung

Bei der Blutbehandlung, insbesondere bei der Durchführung einer Dialysebehandlung, ist es vonnöten, eine Elektrolytlösung, die sogenannte Dialysierflüssigkeit, herzustellen. Diese Dialysierflüssigkeit umspült im Dialysator das Blut eines Patienten und nimmt klein- und mittelmolekulare Substanzen aus dem Blut auf.

Eine Blutbehandlungsmaschine beziehungsweise eine Dialysemaschine benötigt zum Herstellen der Dialysierflüssigkeit gereinigtes Wasser (Permeat), ein Säurekonzentrat und Natriumhydrogenkarbonat, welches entweder in fester Form in einer Kartusche oder in flüssiger Form in einem Kanister enthalten ist, welcher mit der Blutbehandlungsmaschine verbunden wird.

Um der Blutbehandlungsmaschine die zur Herstellung der Dialysierflüssigkeit benötigten Substanzen zur Verfügung zu stellen, können beispielsweise handelsübliche Kanister, welche Säurekonzentrate oder basische Konzentrate beziehungsweise Lösungen enthalten, auf dem Maschinenboden der Blutbehandlungsmaschine platziert werden und über Anschlussleitungen mit der Maschine verbunden werden.

Alternativ besteht die Möglichkeit, genannte Konzentrate aus einer zentralen Quelle direkt in die Maschine zu führen.

Meist werden jedoch die benötigten Konzentrate in Kanistern bereitgestellt. Diese Kanister sind meist mit einem herkömmlichen Deckel verschlossen.

Um die Konzentrate der Blutbehandlungsmaschine zur Verfügung zu stellen, wird der Deckel eines jeden Kanisters jeweils abgeschraubt, und es wird ein runder Gewindeadapter auf den Kanister aufgeschraubt, welcher im Wesentlichen wie ein herkömmlicher Kanisterdeckel mit einer runden Öffnung zur Einführung einer Anschlussleitung ausgestaltet ist. Durch die Öffnung in dem Gewindeadapter kann dann die Anschlussleitung der Blutbehandlungsmaschine in den Kanister gesteckt werden, sodass die Maschine über die Anschlussleitung Konzentrat aus dem Kanister ansaugen kann.

Diese Vorgehensweise hat in der Praxis jedoch einige Nachteile.

Erstens müssen die separaten Gewindeadapter auch separat gelagert werden, wenn die Blutbehandlungsmaschine diese nicht zum Herstellen von Dialysierflüssigkeit benötigt, sodass der Platzbedarf der gesamten Blutbehandlungsanlage steigt.

Zudem können die separaten Adapter während der Lagerung verloren gehen und stehen dann im Bedarfsfalle nicht bereit.

Weiterhin ist die Gefahr von Fehlanschlüssen bei dieser herkömmlichen Vorgehensweise recht hoch, da die verwendeten Gewindeadapter meist die gleiche Einführungsöffnung aufweisen und so z.B. die Anschlussleitung für das Säurekonzentrat fälschlicherweise auch in den Kanister mit der basischen Lösung eingesteckt werden kann.

Bei herkömmlichen Dialysemaschinen oder Blutbehandlungsmaschinen wird ein derartiger Irrtum aber erst dann erkannt, wenn die Maschine das falsche Konzentrat bereits angesaugt hat, wodurch die Patientensicherheit leidet.

### Stand der Technik

Aus dem Stand der Technik sind verschiedene Baugruppen für das Anschließen von Konzentratbehältern an eine Blutbehandlungsmaschine bekannt.

Beispielsweise offenbart JP 2010 184 110 A eine Aufhängungsvorrichtung für eine Flüssigkeitsansaugleitung, welche eine Kappe und einen Abstützabschnitt für die Kappe aufweist.

Weiterhin offenbart die EP 2 730 304 B1 einen Anschluss für Einwegbehälter zur Verwendung für Dialysemaschinen, welcher ein Außengewinde aufweist, welches in einen Anschluss eines Beutels mit vorgemischter Dialyseflüssigkeit eingeschraubt werden kann.

Zudem offenbart die Druckschrift WO 2013/117340 A1 einen Verbinder für einen Behälter mit einer Spikinglösung zur individuellen Anpassung von Dialyselösungen und Konzentraten.

Darüber hinaus offenbart die Druckschrift CN 2019/21175 U einen Deckel, welcher mittels einem Gewindeeingriff auf ein Anschlussstück eines Kanisters aufschraubbar ist.

Ein weiterer Stand der Technik ist die Druckschrift JP1625867 B1, welche eine Vorrichtung für die Gaszufuhr mit zwei verschiedenen Drücken offenbart.

### Kurzbeschreibung der Erfindung

In Anbetracht des Stands der Technik liegt der vorliegenden Erfindung die Aufgabe zugrunde, eine Anschlusseinheit für eine Blutbehandlungsmaschine zum Anschließen der Blutbehandlungsmaschine an einen externen Behälter bereitzustellen, welche die Anzahl der benötigten Einzelteile reduziert und somit eine einfachere Handhabung ermöglicht.

Diese Aufgabe wird gelöst durch eine Anschlusseinheit gemäß einem der Ansprüche 1 bis 10, ein System aus einer Anschlusseinheit und einem externen Behälter gemäß Anspruch 11, sowie durch eine Blutbehandlungsmaschine gemäß einem der Ansprüche 12 bis 14.

Eine erfindungsgemäße Anschlusseinheit für eine Blutbehandlungsmaschine zum Anschließen der Blutbehandlungsmaschine an einen externen Behälter weist auf:
- eine maschinenseitige Leitung, welche dazu ausgelegt ist, mit der Blutbehandlungsmaschine fluidisch verbunden zu werden,
- eine behälterseitige Leitung, welche dazu ausgelegt ist, mit dem Behälter fluidisch verbunden zu werden, und
- einen fest bzw. nicht lösbar mit der maschinenseitigen Leitung und der behälterseitigen Leitung verbundenen Aufsatz, welcher dazu ausgelegt ist, an einem Anschlussstück/Anschlussstutzen des externen Behälters lösbar montiert zu werden, um den externen Behälter fluidisch mit der Blutbehandlungsmaschine zu verbinden.

Der Kern der Erfindung besteht somit darin, den gemäß dem Stand der Technik separat ausgebildeten Gewindeaufsatz / Adapter für den Konzentratkanister mit der Anschlussleitung der Blutbehandlungsmaschine fest vorzugsweise im Sinne von nicht lösbar/abnehmbar zu verbinden, beziehungsweise in diesen zu einer Baugruppe zu integrieren. Durch diese Integration des Gewindeadapters mit der Anschlussleitung der Dialysemaschine kann gewährleistet werden, dass der Adapter nicht separat gelagert werden muss und auch nicht verloren gehen kann, sondern ein integraler / permanenter Teil der Blutbehandlungsanlage, insbesondere der Anschlusseinheit der Blutbehandlungsmaschine ist. Dadurch wird die Handhabbarkeit der Anschlussleitung gegenüber dem Stand der Technik verbessert. Darüber hinaus kann der Platzbedarf der Blutbehandlungsanlage sowie die Anzahl der benötigten Einzelteile reduziert werden. Schließlich ist es dadurch möglich, einen Fehlanschluss von einem falschen Konzentrat an einen falschen Anschluss der Blutbehandlungsmaschine zu verhindern, da jeder Aufsatz unveränderlich einer bestimmten Anschlussleitung zugeordnet ist und daher gleichzeitig die Zuordnung der Anschlussleitung zu einem bestimmten externen Behälter erreicht/gewährleistet wird.

Gemäß einem bevorzugten Aspekt der Erfindung ist der Aufsatz der Anschlusseinheit weiterhin derart ausgestaltet, dass er ausschließlich auf einem Anschlussstück eines externen Behälters eines bestimmten Typs montierbar ist, wobei der Typ des externen Behälter dem Inhalt des Behälters entspricht (beispielsweise ist der Behälter vom Typ "Säurekonzentratbehälter" oder "Basenkonzentratbehälter" etc), wodurch sichergestellt wird, dass ausschließlich der externe Behälter eines bestimmten dem Inhalt des Behälters entsprechenden erwünschten Typs mittels der Anschlusseinheit fluidisch an der Blutbehandlungsmaschine anschließbar ist.

In anderen Worten kann ein bestimmter Aufsatz beispielsweise nur mit einem Säurebehälter oder aber nur mit einem Laugenbehälter verbunden werden. Der bestimmte Aufsatz kann somit nur mit einem jeden Behälter des Typs Säurebehälter oder nur mit einem jeden Behälter des Typs Laugenbehälter verbunden werden.

Hierbei wird vorausgesetzt, dass die einzelnen Behälter eines jeden Typs zumindest bezüglich ihres Anschlussstücks einheitlich ausgestaltet sind. Hierbei wird weiterhin zugrunde gelegt, dass die Anschlussstücke eines Behälters mit Säure und eines Behälters mit Lauge voneinander verschieden sind. Der Typ eines Behälters bezeichnet demgemäß den Typ "Säurebehälter" oder "Laugenbehälter" etc. Der Typ des externen Behälters entspricht somit dem Inhalt des Behälters und bestimmt die Ausgestaltung des Anschlussstücks des externen Behälters, welches als Montagestück für den Aufsatz einer Anschlusseinheit einer Blutbehandlungsmaschine dient.

Durch diese eindeutige Zuordnung eines Aufsatzes einer Anschlusseinheit nach dem Schlüssel-und-Schloss-Prinzip zu einem besonderen Typ von externen Behälter, welcher einem definierten Inhalt des Behälters entspricht, kann verhindert werden, dass irrtümlicherweise ein Behälter mit Säure an einen Anschluss angeschlossen wird, welcher eigentlich an einen Behälter mit Base angeschlossen werden sollte. Ein für einen Säurebehälter vorgesehener Aufsatz kann aufgrund seiner geometrischen Ausgestaltung nämlich gar nicht an einem Basenbehälter montiert werden. Dadurch wird die Patientensicherheit signifikant erhöht.

Gemäß einem Aspekt der Erfindung ist der Aufsatz der Anschlusseinheit weiterhin dazu ausgelegt, dass er durch Formschluss oder Reibschluss an dem Anschlussstück des externen Behälters vorherbestimmten Typs gehalten wird, wenn der Aufsatz auf dem Anschlussstück montiert ist.
Konkret kann der Aufsatz hierbei beispielsweise als ein formelastischer Balg beziehungsweise als ein Gummibalg ausgestaltet sein, welcher über das Anschlussstück des externen Behälters stülpbar beziehungsweise überziehbar ist.

Ein derartiger formelastischer Aufsatz der Anschlusseinheit kann einen im Wesentlichen elastisch verformbaren zylindrischen Grundkörper aufweisen, dessen Durchmesser im Ruhezustand kleiner ist als der Durchmesser des Anschlussstücks des externen Behälters, und der daher nur in gedehntem Zustand auf das Anschlussstück des externen Behälters aufgebracht werden kann.

Alternativ kann der Reibschluss aber auch dadurch verwirklicht sein, dass der Aufsatz als eine formstabile Kappe ausgeformt ist, welcher eine bevorzugt glatte Innenseite hat und im Wesentlichen spielfrei auf ein korrespondierendes Anschlussstück des Behälters mit einer glatten Außenseite des Anschlussstücks aufgesteckt werden kann, wobei ein Reibschluss zwischen der Innenseite des kappenförmigen Aufsatzes und der Außenseite des Anschlussstückes des externen Behälters den Aufsatz an dem Behälter hält.

Gemäß einem weiteren Aspekt der Erfindung kann der Aufsatz auch um die behälterseitige Leitung drehbar und als formstabile Kappe ausgestaltet sein, wobei die formstabile Kappe vorzugsweise ein Innengewinde aufweist, welches dazu ausgelegt ist, mit einem entsprechenden Außengewinde des Anschlussstücks des externen Behälters des vorherbestimmten Typs in Eingriff gebracht zu werden. Der Aufsatz wird dann formschlüssig an dem Anschlussstück des externen Behälters gehalten.

Alternativ sind auch andere Arten des Formschlusses denkbar, wie beispielsweise ein Bajonettverschluss, eine Verankerung mittels in Aussparungen eingreifenden Rastarmen oder über einen Hinterschnitt. Der Formschluss bietet den Vorteil einer besonders sicheren und stabilen Verankerung des Aufsatzes der Anschlusseinheit an dem Anschlussstück des externen Behälters.

Gemäß einem weiteren Aspekt der Erfindung weist die Anschlusseinheit zusätzlich ein Verstärkungselement auf, welches sich im Bereich des Aufsatzes und über den Bereich des Aufsatzes hinaus hin zu einem behälterseitigen Ende der behälterseitigen Leitung der Anschlusseinheit für einen definierten Längenabschnitt entlang der behälterseitigen Leitung erstreckt.

In anderen Worten wird durch das Verstärkungselement die behälterseitige Leitung im Bereich des Aufsatzes und etwas darüber hinaus verstärkt. Diese Verstärkung vereinfacht die Handhabung der Anschlusseinheit sowie die Montage der Anschlusseinheit mittels des Aufsatzes an einem externen Behälter, da die behälterseitige Leitung nicht willkürlich abknickt.

Gemäß einem anderen Aspekt der Erfindung ist an der behälterseitigen Leitung und / oder an dem Verstärkungselement, vorzugsweise benachbart zu dem Aufsatz, mindestens ein Begrenzungselement vorgesehen, welches eine axiale Bewegung des Aufsatzes entlang der Längsachse der behälterseitigen Leitung und oder des Verstärkungselements begrenzt.

Ist ein Verstärkungselement vorgesehen, so ist das mindestens eine Begrenzungselement vorzugsweise an dem Verstärkungselement angeordnet. Ist kein Verstärkungselement vorgesehen, so ist das mindestens eine Begrenzungselement an der behälterseitigen Leitung vorgesehen.

Das Begrenzungselement hat vorzugsweise die Form eines Vorsprungs beziehungsweise eines Absatzes, oder auch eines ringförmigen umlaufenden Vorsprungs, auf welchem der Aufsatz aufliegt, wodurch ein Verschieben entlang der Längsachse des Verstärkungselements verhindert wird.

Konkret kann das Begrenzungselement auch als ein Gummiring ausgeformt sein, welcher auf das Verstärkungselement aufgeschoben ist. Bevorzugt wird der Aufsatz sozusagen auf der einen Seite durch das mindestens eine Begrenzungselement und auf der anderen Seite durch ein Griffbauteil der Anschlusseinheit an einer axialen Bewegung in beide Richtungen gehindert.

Gemäß einem weiteren Aspekt der Erfindung weist die Anschlusseinheit weiterhin mindestens ein Dichtelement auf, welches an dem Verstärkungselement und oder der maschinenseitigen Leitung angeordnet ist, und welches dazu ausgelegt ist, einen Zwischenraum zwischen der Außenseite des Verstärkungselements beziehungsweise der maschinenseitigen Leitung und einer Innenseite eines Anschlusses der Blutbehandlungsmaschine fluidisch abzudichten, wenn das Verstärkungselement beziehungsweise die behälterseitige Leitung in den Anschluss / die Aufnahme der Blutbehandlungsmaschine eingeführt ist. In anderen Worten ist mindestens ein, sind vorzugsweise zwei, Dichtelement(e) 5a,5b vorgesehen, welche(s) an dem Anschlussnippel 8a vorzugsweise im Bereich der Verstärkung angeordnet sind/ist und welche(s) dazu ausgelegt sind/ist, einen Zwischenraum zwischen der Außenseite des Anschlussnippels 8a vorzugsweise der Verstärkung und einer Innenseite einer Aufnahme der Blutbehandlungsmaschine 21 und/oder des Anschlussstutzens 9 des externen Behälters fluidisch abzudichten, wenn das Verstärkungselement 8 in den Anschluss der Blutbehandlungsmaschine 21 oder den Anschlussstutzen des Behälters eingeführt ist.

In der Praxis wird die behälterseitige Leitung beziehungsweise das Verstärkungselement während eines Spülvorgangs oder zur Lagerung, falls die Anschlussleitung nicht benötigt wird, in den Anschluss / der Aufnahme der Blutbehandlungsmaschine eingeführt beziehungsweise verstaut / verräumt.

Gemäß einem weiteren Aspekt der Erfindung weist die Anschlusseinheit weiterhin eine Freigabevorrichtung auf, welche einen Fluidfluss zwischen der Anschlusseinheit und dem externen Behälter nur dann freigibt, wenn der Aufsatz der Anschlusseinheit korrekt in einer vorherbestimmten Montageposition an dem Anschlussstück des externen Behälters montiert ist. Durch die Freigabevorrichtung wird der Fluidfluss vorzugsweise nur dann freigegeben, wenn die Anschlusseinheit korrekt an der Blutbehandlungsmaschine angebracht ist (zum Beispiel während einer Desinfektion oder eines Spülvorgangs). Andernfalls könnte beispielsweise während einer Heißdesinfektion heißes Desinfektionsmittel aus der Maschine strömen, wodurch die Verletzungsgefahr für einen Benutzer erhöht wird.

Die Freigabevorrichtung kann hierbei durch die Anschlusseinheit allein oder gemeinsam durch die Anschlusseinheit und den externen Behälter ausgestaltet werden.

Konkret kann die Freigabevorrichtung beispielsweise ein Reedkontakt sein, bei dem der Aufsatz mit einem Reedkontakt und das Anschlussstück des externen Behälters mit einem Magneten ausgestattet ist. Erst wenn der Kontakt durch die Nähe des Magneten geschlossen ist, kann die Anschlusseinheit ein entsprechendes Ventil öffnen und eine Zufuhr des Konzentrats an die Blutbehandlungsmaschine freigeben.

Weiterhin kann auch in der Anschlusseinheit ein kapazitiver Sensor verbaut sein, der auf ein entsprechendes Material des externen Behälters reagiert und somit bestimmt, ob der Aufsatz der Anschlusseinheit korrekt auf dem Anschlussstück des Behälters montiert worden ist.

Alternativ oder zusätzlich ist auch die Verwendung einer Reflexionslichtschranke denkbar, welche bestimmt, dass das Anschlussstück des Behälters korrekt in den Aufsatz der Anschlussleitung eingeführt ist, und erst dann einen Fluidfluss zwischen der Anschlusseinheit, dem Behälter und der Blutbehandlungsvorrichtung freigibt.

Auf diese Weise wird verhindert, dass die externen Behälter offengelassen werden, was das Risiko einer potenziellen Kontamination durch das Eindringen von Partikeln aus der Umgebungsluft birgt, wodurch sichergestellt wird, dass nur dann Konzentrat über die behälterseitige Leitung angesaugt wird, wenn die Anschlusseinheit fest mit dem externen Behälter verbunden ist.

Ein anderer Aspekt der vorliegenden Erfindung betrifft eine Blutbehandlungsmaschine mit einer erfindungsgemäßen Anschlusseinheit, welche über die maschinenseitige Leitung der Anschlusseinheit mit einem vorherbestimmten Anschluss der Blutbehandlungsmaschine fluidisch verbunden ist.

Vorzugsweise ist die Anschlusseinheit über die maschinenseitige Leitung der Anschlusseinheit permanent mit der Blutbehandlungsmaschine gekoppelt. Durch diese feste Verbindung zwischen der Anschlusseinheit und der Blutbehandlungsmaschine und die ausschließliche Verbindbarkeit zwischen dem Aufsatz der Anschlusseinheit und nur dem externen Behälter, welcher das korrekte Konzentrat (Säure oder Base) enthält, kann sichergestellt werden, dass die Blutbehandlungsmaschine über jeden Anschluss nur das korrekte Konzentrat ansaugen kann. Somit wird die Sicherheit bei der Herstellung von Dialyseflüssigkeit erhöht.

Ein weiterer Aspekt der Erfindung betrifft eine Blutbehandlungsmaschine mit einer Mehrzahl an Anschlusseinheiten gemäß der vorliegenden Erfindung, welche jeweils mit einem jeweiligen zugehörigen und vorherbestimmten Anschluss der Blutbehandlungsmaschine fluidisch verbunden sind. Jeder Aufsatz einer jeden Anschlusseinheit der Mehrzahl an Anschlusseinheiten ist derart ausgestaltet, dass der Aufsatz nur an dem Anschlussstück eines externen Behälters eines jeweiligen vorherbestimmten Typs (beispielsweise Säurebehälter oder Basenbehälter) lösbar montierbar ist, wobei der Typ des Behälters dem Inhalt des Behälters entspricht.

In anderen Worten weist die Blutbehandlungsmaschine somit beispielsweise eine Anschlusseinheit zur Zuführung von Säurekonzentrat, welche einen Aufsatz umfasst, welcher nur an Behältern mit Säurekonzentrat montierbar ist und eine Anschlusseinheit für die Zuführung von basischem Konzentrat auf, welche einen Aufsatz aufweist, welcher nur an einem Behälter mit Basenkonzentrat montierbar ist.

Weiterhin ist es auch vorteilhaft, dass gemäß einem anderen Aspekt der Erfindung mindestens eine, aber vorzugsweise eine jede der Anschlusseinheiten der Blutbehandlungsmaschine zusätzlich eine Freigabevorrichtung aufweist, welche einen Fluidfluss zwischen der Blutbehandlungsmaschine und dem jeweiligen zugehörigen externen Behälter nur dann freigibt, wenn der Aufsatz der jeweiligen Anschlusseinheit korrekt in einer vorherbestimmten Montageposition an dem Anschlussstück des jeweiligen externen Behälters montiert ist.

In anderen Worten wird durch die Freigabevorrichtung eine weitere Sicherheitsebene eingezogen, welche gewährleistet, dass für einen Fluidaustausch zwischen der Blutbehandlungsmaschine und dem externen Behälter nicht nur jeweils der korrekte Typ von externem Behälter (Säurebehälter oder Basenbehälter) an der jeweils korrekten Anschlusseinheit über den Aufsatz angeschlossen ist, sondern das der Fluidfluss auch nur dann freigeschaltet wird, wenn der Aufsatz auch wirklich korrekt und fest an dem jeweiligen externen Behälter montiert ist.

So kann es beispielsweise nicht geschehen, dass ein Behälter dessen Aufsatz der Anschlusseinheit nur locker auf das Anschlussstück des Behälters aufgesteckt ist, über eine längere Zeit stehen gelassen wird, während die Blutbehandlungsmaschine Dialysierflüssigkeit aufbereitet, ohne das Personal dies bemerkt.

Viel mehr wird direkt bei der Montage der Anschlusseinheit an dem Anschlussstück des externen Behälters einem Benutzer angezeigt, dass der Aufsatz nicht korrekt an dem Anschlussstück des externen Behälters montiert ist. So werden Kontaminationen der Konzentrate beziehungsweise des Inhalts der Behälter verhindert, da verhindert wird, dass die Behälter nur in locker geschlossenem Zustand über längere Zeit der Außenluft ausgesetzt sind.

Ein weiterer Aspekt der Erfindung betrifft zudem ein System aus einer Anschlusseinheit gemäß der vorliegenden Erfindung und einem externen Behälter, wobei das System vorzugsweise eine Freigabevorrichtung aufweist, welche einen Fluidfluss zwischen der Anschlusseinheit und dem externen Behälter nur dann freigibt, wenn der Aufsatz der Anschlusseinheit korrekt in einer vorherbestimmten Montageposition an dem Anschlussstück des externen Behälters montiert ist.

Mit einem derartigen System können bereits bestehende Blutbehandlungsmaschinen nachgerüstet werden.

### Figurenbeschreibung

Weitere Vorteile und Merkmale der vorliegenden Erfindung ergeben sich aus der folgenden Beschreibung bevorzugter Ausführungsformen mit Bezug auf die zugehörigen Figuren. Hierbei zeigt:
Figur 1 eine erfindungsgemäße Anschlusseinheit für eine Blutbehandlungsmaschine zum Anschließen der Blutbehandlungsmaschine an einen externen Behälter,
Figur 2a eine Anschlusseinheit für eine Blutbehandlungsmaschine zum Anschließen der Blutbehandlungsmaschine an einen externen Behälter in einer Stellung während eines Spülvorgangs der Blutbehandlungsmaschine, in welchem die behälterseitige Leitung und das Verstärkungselement in einer Aufnahme / Anschluss der Blutbehandlungsmaschine eingeführt sind;
Fig. 2b eine Variante der Anordnung aus Fig. 2a, in welcher die Blutbehandlungsmaschine ein Anschlussstück mit Außengewinde aufweist;
Figur 3 eine erfindungsgemäße Anschlusseinheit für eine Blutbehandlungsmaschine zum Anschließen der Blutbehandlungsmaschine an einen externen Behälter, welche auf dem externen Behälter in einer korrekten Montageposition montiert ist, und
Figur 4 eine Blutbehandlungsmaschine mit einer erfindungsgemäßen Anschlusseinheit.

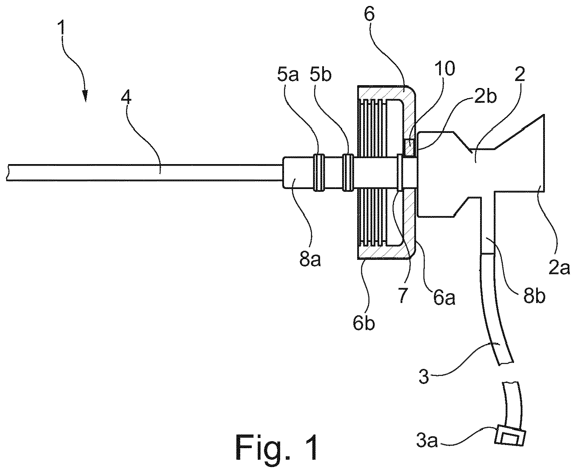

Figur 1 zeigt eine Anschlusseinheit 1 für eine Blutbehandlungsmaschine zum Anschließen der Blutbehandlungsmaschine an einen (in Fig. 1 nicht gezeigten) externen Behälter mit einer maschinenseitigen Leitung (Verbindungsleitung) 3, welche dazu ausgelegt ist mit der Blutbehandlungsmaschine fluidisch verbunden zu werden, einer behälterseitigen Leitung (Saugleitung/Saugrohr) 4, welche dazu ausgelegt ist, mit dem externen Behälter fluidisch verbunden zu werden bzw. in einen Behälter eingeführt zu werden, und einem fest mit der maschinenseitigen Leitung 3 und der behälterseitigen Leitung 4 verbundenen Aufsatz 6, welcher dazu ausgelegt ist, an einem Anschlussstück/Anschlussstutzen des externen Behälters lösbar montiert (auf-/eingeschraubt oder aufgestülpt/eingesteckt) zu werden, um den externen Behälter fluidisch mit der Blutbehandlungsmaschine zu verbinden.

Zur besseren Handhabung weist die Anschlusseinheit 1 eine Art Stecker 2 in Form eines L-Stücks vorzugsweise aus einem starren (Kunststoff-)Material auf, an dem weiterhin ein Griff 2a an/ausgeformt ist, mit welchem ein Benutzer die Anschlusseinheit/den Stecker 2 handhaben kann, und welcher bevorzugt ergonomisch an die Hand eines Benutzers angepasst ist. Der Stecker 2 hat zwei fluidisch miteinander verbundene Anschlussnippel 8a, 8b, die im Wesentlichen rechtwinklig zueinander ausgerichtet sind und auf welche die maschinenseitige (Verbindungs-)Leitung 3 und die behälterseitige (Saug-)Leitung 4 fluiddicht aufgesteckt sind. Alternativ können die Leitungen 3, 4 aber auch in die Anschlussnippel 8a, 8b bzw. in den Stecker 2 eingesteckt sein.

Die zwei fluidisch miteinander verbundenen Anschlussnippel 8a, 8b des Steckers sind bei der vorliegenden beispielhaften Ausführungsform zwar rechtwinklig zueinander angeordnet, die vorliegende Erfindung ist aber nicht auf diesen Winkel beschränkt und die zwei Anschlussnippel 8a, 8b können auch in einem beliebigen anderen Winkel zueinander angeordnet sein.

Zudem können die Anschlussnippel 8a, 8b auch jeweils mit den Leitungen 3 und 4 fest verbunden sein, anstatt mit den Leitungen 3 und 4 über eine Steckverbindung (durch einstecken bzw. aufstecken) verbunden zu sein.

Schließlich bildet der Stecker 2 auf seiner dem Behälter zugewandten Seite einen Anschlag vorzugsweise in Form eines Kragens 2b, der radial über den behälterseitigen Anschlussnippel 8a ringförmig vorragt. Insbesondere der behälterseitige Anschlussnippel 8a hat an seinem proximalen Endabschnitt bevorzugt eine Art Verstärkung, d.h. dessen Außendurchmesser ist in seinem proximalen Endabschnitt etwas größer als an seinem distalen Endabschnitt.

Die Leitungen 3, 4 sind starre oder flexible Leitungen. Die maschinenseitige Leitung 3 ist vorzugsweise permanent mit der Blutbehandlungsmaschine fluidisch verbunden. Es ist aber auch alternativ möglich, die maschinenseitige Leitung 3 an ihrem der Blutbehandlungsmaschine zugewandten Ende mit einem Anschlussstück/Kupplung 3a zu versehen, die vorzugsweise an eine speziell hierfür ausgebildete Kupplung an der Blutbehandlungsmaschine anschließbar ist.

Die behälterseitige Leitung 4 ist in eine Öffnung des externen Behälters/Kanisters eingeführt bzw. einführbar und steht vorzugsweise in direkten Kontakt zu dem im externen Behälter/Kanister enthaltenen Konzentrat.

Die Länge der Leitungen 3, 4 kann jeweils variabel sein, was den Vorteil bietet, Konzentrate anzusaugen, die sich in externen Behältern befinden, welche von der üblichen Kanistergeometrie/-größe beziehungsweise Behältergeometrie/-größe abweichen. So können beispielsweise auch Kanister leergesaugt werden, die höher als übliche Kanister ausfallen, ohne dass Konzentratreste im Kanister verbleiben.

Bei der vorliegenden Ausführungsform ist der Aufsatz 6 als ein kappenförmiges, formstabiles Bauteil ausgebildet, welches einen zentral mit einer Durchgangsbohrung ausgebildeten Kappenboden 6a und eine ein Innengewinde ausbildende Umfangswand 6b aufweist, welches dazu ausgelegt ist, mit einem Außengewinde eines Anschlussstücks/Anschlussstutzens des externen Behälters in Eingriff zu treten.

Die Durchgangsbohrung im Kappenboden 6a des Aufsatzes 6 hat im Wesentlichen einen Inndurchmesser, der ein Einschieben des behälterseitigen Anschlussnippels 8a des Steckers 2 erlaubt. Alternativ ist es aber auch möglich, den Stecker 2 sowie den Aufsatz 6 (stoff-)einstückig miteinander auszuformen. Der behälterseitige Anschlussnippel 8a ist ferner so dimensioniert, dass es sich entlang der behälterseitigen Leitung 4 im Bereich des Aufsatzes 6 und darüber hinaus für eine definierte Länge hin zu dem behälterseitigen Ende der behälterseitigen Leitung 4 (nicht gezeigt) erstreckt.

In der vorliegenden Ausführungsform hat der Anschlussnippel 8a im Wesentlichen die Form eines Rohrs, auf welches die behälterseitige Leitung 4 aufgesteckt ist, um so einen Fluiddurchlass zu der maschinenseitigen Leitung 3 zu schaffen.

Weiterhin weist die Anschlusseinheit 1 gemäß der vorliegenden Ausführungsform ein Begrenzungselement 7 vorzugsweise in Form einer ringartigen Wellensicherung auf, welches an dem Anschlussnippel 8a vorzugsweise im Bereich seiner Verstärkung vorgesehen ist, und in dieser Ausführungsform als ein den Anschlussnippel 8a umlaufender und auf den Anschlussnippel 8a aufgeschobener Gummiring ausgeführt ist. Alternativ und / oder zusätzlich können jedoch auch radiale Vorsprünge an dem Anschlussnippel 8a vorgesehen sein, welche die Funktion des Begrenzungselements übernehmen.

Wie in der Figur 1 gezeigt, ist das Begrenzungselement 7 in unmittelbarer Nachbarschaft des kappenförmigen Aufsatzes 6 insbesondere von dessen Kappenboden 6a angeordnet und zwar auf der dem behälterseitigen Ende des Anschlussnippels 8a zugewandten Seite des Aufsatzes 6 bzw. des Kappenbodens 6a. In anderen Worten wird der Aufsatz 6 bzw. dessen Kappenboden 6a somit zwischen dem Begrenzungselement 7 und dem Anschlag 2b eingeklemmt, und somit in axialer Richtung fixiert.

Wie zudem in Figur 1 ersichtlich, weist die Anschlusseinheit 1 an dem Anschlussnippel 8a vorzugsweise im Bereich seiner Verstärkung weiterhin zwei Dichtelemente (Gummiringe) 5a und 5b auf, welche in axialer Richtung des Anschlussnippels 8a voneinander beabstandet an/auf diesem angeordnet sind und so eine Art Doppeldichtung bilden. Im Fall, dass die behälterseitige Leitung 4 über den Anschlussnippel 8a gestülpt/gezogen ist, sind die beiden Dichtelemente 5a, 5b so dimensioniert, dass sich radial über den Außenradius der übergestülpten Leitung 4 erstrecken.

In der vorliegenden Ausführungsform sind die Dichtelemente 5a und 5b als Dichtringe ausgestaltet. Die Anzahl der Dichtelemente ist beliebig, zumindest aber ein Dichtelement muss vorhanden sein. Die Dichtelemente 5a und 5b sind in ihrer Position entlang des Anschlussnippels 8a dadurch fixiert, dass sie jeweils zwischen zwei den Anschlussnippel 8a ringförmig umlaufenden Vorsprüngen (Wellenringen) eingebettet sind, sodass sie sich nicht in axialer Richtung des Anschlussnippels 8a aus ihrer vorherbestimmten Position wegbewegen können.

Zudem weist die Anschlusseinheit 1 eine Freigabevorrichtung 10 auf (in Fig. 1 nur andeutungsweise dargestellt), welche gewährleistet, dass der Fluidaustausch zwischen der Blutbehandlungsmaschine und dem externen Behälter über die Anschlusseinheit 1 erst dann erfolgt, wenn der Aufsatz 6 der Anschlusseinheit 1 korrekt (und vollständig) an dem Anschlussstück des externen Behälters montiert ist.

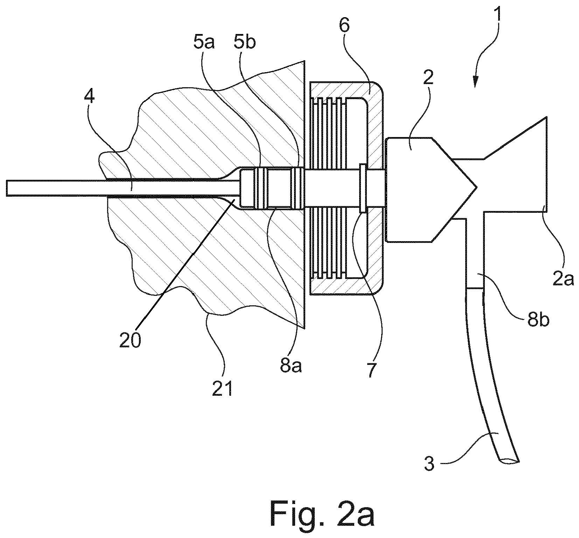

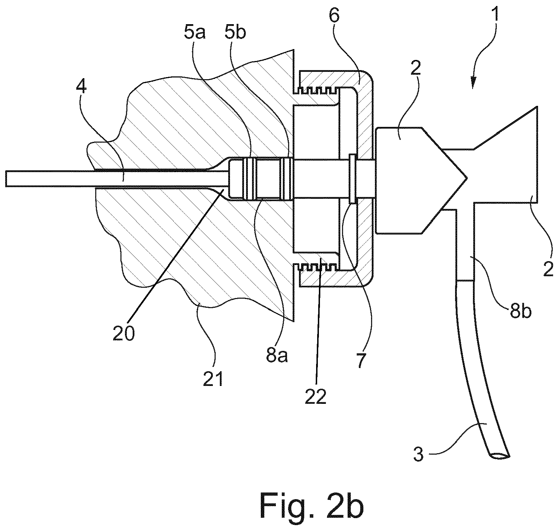

Figur 2 zeigt die erfindungsgemäße Anschlusseinheit 1 aus Figur 1, welche während eines Spülvorgangs der Blutbehandlungsmaschine mit dieser eine Spülbrücke bildet.

Konkret sind die behälterseitige Leitung 4 und der Anschlussnippel 8a in einer entsprechend geformten Aufnahmeöffnung 20 der Blutbehandlungsmaschine 21 eingeführt/eingesteckt. Der Anschlussnippel 8a ist in diesem Zustand nur teilweise in der Aufnahmeöffnung 20 der Blutbehandlungsmaschine 21 eingeführt, bis zu dem Punkt, zu dem der Aufsatz 6 auf der Oberfläche der Blutbehandlungsmaschine 21 zu liegen kommt, wodurch ein weiteres Einführen des Anschlussnippels 8a in die Aufnahmeöffnung 20 der Blutbehandlungsmaschine 21 verhindert wird.

Wie in der Figur 2a gezeigt, dichten in diesem Zustand die Dichtelemente 5a und 5b im Verstärkungsbereich des Anschlussnippels 8a den Zwischenraum zwischen der Außenseite des Anschlussnippels 8a und einer Innenseite der Aufnahmeöffnung 20 der Blutbehandlungsmaschine 21 fluidisch ab, wodurch ein ungewollter Fluidaustritt, beispielsweise von Spülflüssigkeit, aus der Aufnahmeöffnung 20 der Blutbehandlungsmaschine 21 nach außen verhindert wird.

Die in Fig. 2 gezeigte Position ist die bevorzugte Position der Anschlusseinheit 1, wenn diese nicht benötigt wird, oder sich im Rahmen eines maschinenseitigen Spülvorgangs beziehungsweise Desinfektion in der Blutbehandlungsmaschine befinden muss.

In dieser Position liegt, wie in Figur 2a gezeigt, der Aufsatz 6 auf der äußeren Maschinenoberfläche auf. Gleichzeitig dichten die Dichtelemente 5a, 5b den Zwischenraum zwischen dem Anschlussnippel 8a und der Blutbehandlungsmaschine 21 ab, sodass keine Flüssigkeit aus der Maschine 21 an den Dichtelementen 5a, 5b vorbei nach außen gelangen kann. So wird sichergestellt, dass auch die maschinenseitige Leitung 4 bei dem Spülvorgang der Blutbehandlungsmaschine 21 gespült beziehungsweise desinfiziert werden kann.

Dies ist äußerst wichtig, da eine äußere Desinfektion der Komponenten des Aufsatzes 6, des Begrenzungselements 7 und des Griffteils 2 nicht vorgesehen ist, sodass die Dichtelemente 5a und 5b auch dafür unerlässlich sind, zu verhindern, dass etwaige Partikel von außen durch die Spülaufnahme in die Spülflüssigkeit gelangen und somit in der gesamten Blutbehandlungsmaschine 21 zirkuliert werden.

Alternativ oder zusätzlich ist es möglich, dass die Anschlusseinheit 1 in dieser Position während des Spülvorgangs der Blutbehandlungsmaschine 21 über den Aufsatz 6 an der Blutbehandlungsmaschine 21 befestigt/verschraubt wird.

Bei einer derartigen in Fig. 2b gezeigten Ausführungsform weist die Maschinenoberfläche ein entsprechendes Anschlussstück 22 auf, auf welches der Aufsatz 6 aufgeschraubt wird. Das Anschlussstück der Blutbehandlungsmaschine 21 muss somit ein Außengewinde aufweisen.

Bei dieser Variante wären die Dichtelemente 5a und 5b nicht unbedingt vonnöten, da durch den Gewindeeingriff des Anschlussstückes der Blutbehandlungsmaschine und des Aufsatzes 6 die Anschlusseinheit 1 sicher an der Blutbehandlungsmaschine 21 gehalten wird und zudem der Kreislauf der Spülflüssigkeit sicher von äußeren Kontaminanten abgeschirmt wäre.

Diese Ausführungsform würde es ermöglichen, auch die Innenseite des Aufsatzes 6 und das Begrenzungselement 7 bei einer automatischen maschinenseitigen Desinfektion beziehungsweise einem Spülvorgang mit zu desinfizieren beziehungsweise zu spülen. Bei einer derartigen Ausführungsform sind die Dichtelemente nicht vollständig dichtend ausgestaltet oder es ist mindestens eine zusätzliche Öffnung in der Oberfläche der Blutbehandlungsmaschine 21 innerhalb des Anschlussstücks vorgesehen, um Spülflüssigkeit in das Innere des Aufsatzes 6 einzuspeisen.

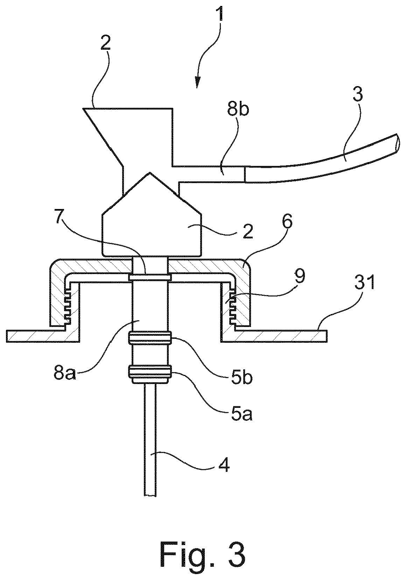

Figur 3 zeigt die erfindungsgemäße Anschlusseinheit der Figuren 1 und 2, in einer Position, in welcher die Anschlussleitung mit einem zu dem Aufsatz 6 passenden externen Behälter bzw. dessen Anschlussstück 9 formschlüssig verbunden ist.

Der externe Behälter weist wie vorliegend gezeigt ein Anschlussstück 9 mit einem Außengewinde auf, welches sich mit dem Innengewinde des Aufsatzes 6 in Eingriff befindet.

In dieser Ausführungsform ist der Aufsatz 6 drehbar um den proximalen Verstärkungsabschnitt des Anschlussnippels 8a und die behälterseitige Leitung 4 gelagert, sodass bei der Montage der Anschlusseinheit 1 an dem Behälter 31 der Anschlussnippel 8a und die behälterseitige Leitung 4 einfach in den Behälter 31 eingeführt werden können und daraufhin der Aufsatz 6 auf das Anschlussstück 9 aufgeschraubt werden kann, ohne das die mit der Blutbehandlungsmaschine 21 verbundene maschinenseitige Leitung 3 verdreht wird.

Wird der Aufsatz 6 alternativ als formelastischer (Gummi-)Balg ausgeführt, so wird der Aufsatz 6 einfach über das Außengewinde bzw. den Anschlussstutzen (Anschlussstück) 9 des Behälters 31 übergestülpt. Vorteilhafterweise besteht ein derartiger Aufsatz 6 aus einem säurebeständigen, formelastischen Material, wie beispielsweise Silikon. Allgemein ist es von Vorteil, wenn die Komponenten der Anschlusseinheit 1, insbesondere der Aufsatz 6 aus einem Kunststoff gefertigt sind.

Die Erfindung sieht vor, dass der Durchmesser des Aufsatzes 6 abhängig von dem Typ von Behälter eingestellt wird, mit dessen Anschlussstück der Aufsatz 6 verbunden werden soll. Um beispielsweise zu vermeiden, dass es zu einer Fehlverbindung zwischen den Anschlüssen für saures Konzentrat und basisches Konzentrat und den Behältern für saures Konzentrat und basisches Konzentrat kommt, kann der Durchmesser des Aufsatzes 6, welcher für eine Verbindung mit dem Behälter für das saure Konzentrat vorgesehen ist, größer oder kleiner eingestellt werden, als der Durchmesser des Aufsatzes einer anderen Anschlusseinheit, welche dafür vorgesehen ist, mit einem Behälter von basischem Konzentrat verbunden zu werden.

Dies setzt voraus, dass die entsprechenden Außengewinde der jeweiligen zugehörigen Behälter mit Säurekonzentrat beziehungsweise Basenkonzentrat ebenfalls analog zu der Ausgestaltung des Aufsatzes 6 einen unterschiedlichen Durchmesser aufweisen, sodass der jeweilige Aufsatz und der jeweilige zugehörige Typ von Behälter ausschließlich miteinander nach dem Schlüssel-Schloss-Prinzip verbindbar sind. Zusätzlich dazu ist eine Farbcodierung des Aufsatzes 6 oder irgendeines anderen Bauteils der Anschlusseinheit beziehungsweise des jeweiligen zugehörigen Typs von Behälter möglich.

Beispielsweise können Säurekonzentrate stets in roten Behältern aufbewahrt werden, auf welche jeweils nur ein rot eingefärbter Aufsatz aufschraubbar ist. Dies erleichtert einem Benutzer weiterhin das korrekte Montieren des korrekten Aufsatzes an dem jeweils zugehörigen Behälter.

Weiterhin ist es denkbar, die Gewinderichtung unterschiedlich zu gestalten, sodass beispielsweise für die Verbindung eines Adapters 6 mit dem zugehörigen Behälter für saures Konzentrat ein Rechts- beziehungsweise Linksgewinde verwendet wird und für die Verbindung eines zugehörigen anderen Aufsatzes 6 mit einem Behälter für basisches Konzentrat das andere von dem Links- beziehungsweise Rechtsgewinde verwendet wird. Somit wäre ebenso eine Fehlmontage eines Aufsatzes auf einem nicht vorgesehenen Typ von Behälter ausgeschlossen.

Da offene Konzentratkanister das Risiko einer potentiellen Verunreinigung durch das mögliche Eindringen von Partikeln von außen in den Behälter bergen, kann es, um sicherzustellen, dass nur dann ein Konzentrat über die Anschlusseinheit 1 durch die Blutbehandlungsmaschine angesaugt wird, wenn die Anschlusseinheit 1 fest mit dem Behälter verbunden ist, vorgesehen sein, die Anschlusseinheit 1 beziehungsweise den Aufsatz 6 mit einer Freigabevorrichtung 10 auszustatten, wie diese eingangs dieser Beschreibung bereits kurz erwähnt wurde.

Beispielsweise kann der Gewindeaufsatz 6 mit einem Reedkontakt ausgestattet sein und ein zugehöriger Typ von Behälter kann mit einem Magneten ausgestattet sein. Erst wenn der Kontakt durch die Nähe des Magneten geschlossen wird, würde die Freigabevorrichtung 10 einen Fluidfluss zwischen der Blutbehandlungsmaschine 21 und dem Behälter 31 freigeben, indem ein entsprechendes Ventil beispielsweise innerhalb des Steckers 2 oder des Behälters 31 oder der Blutbehandlungsmaschine 21 geöffnet wird, was eine Zufuhr des entsprechenden Konzentrats aus dem Behälter ermöglicht. Weiterhin wäre als Freigabevorrichtung 10 auch ein kapazitiver Sensor oder eine Reflexionslichtschranke denkbar, die durch die Verwendung eines Senders und eines Empfängers in der Anschlusseinheit 1 die korrekte Montage des Behälters 31 an der Anschlusseinheit 1 anzeigt.

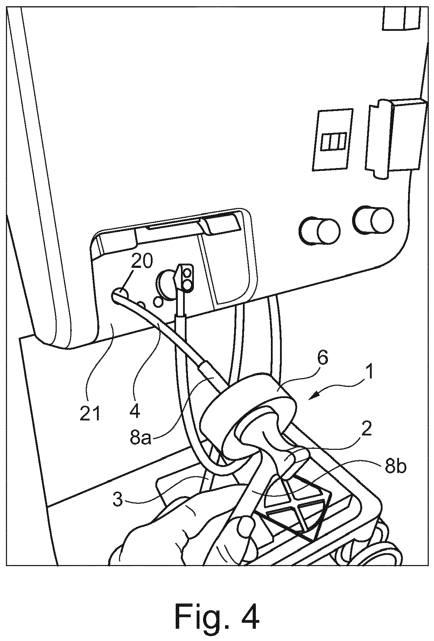

Figur 4 zeigt eine Blutbehandlungsmaschine 21, im vorliegenden Fall eine Dialysemaschine, welche mit einer erfindungsgemäßen Anschlusseinheit 1 ausgestattet ist. Die erfindungsgemäße Anschlusseinheit 1 ist hierbei über die maschinenseitige Leitung 3 der Anschlusseinheit 1 an der Blutbehandlungsmaschine 21 angebracht.

Für einen Spülvorgang kann die Anschlusseinheit 1 an der Blutbehandlungsmaschine 21 angebracht werden, indem zumindest auch die behälterseitige Leitung 4 und ggf. auch das Verstärkungselement 8 in einen entsprechende Aufnahme der Blutbehandlungsmaschine 21 eingesteckt wird.

Die Anschlusseinheit 1 und somit auch der der Anschlusseinheit 1 zugehörige Aufsatz 6 ist der Blutbehandlungsmaschine 21 permanent fest zugeordnet. Es ist daher kein separater Aufsatz vorgesehen, der separat gelagert werden muss und verloren gehen kann. Daher wird der Aufbau der Blutbehandlungsanlage vereinfacht.

## Patentansprüche

1. Anschlusseinheit (1) für eine Blutbehandlungsmaschine (21) zum Anschließen der Blutbehandlungsmaschine (21) an einen externen Behälter (31), mit
einer maschinenseitigen Leitung (3), welche dazu ausgelegt ist, mit der Blutbehandlungsmaschine (21) fluidisch verbunden zu werden,
einer behälterseitigen Leitung (4), welche dazu ausgelegt ist, mit dem Behälter (31) fluidisch verbunden zu werden,
einem vorzugsweise steckerförmigen Anschlussstück (2), welches zwischen die maschinenseitige und behälterseitige Leitungen (3, 4) zwischengeschaltet ist und
einem Aufsatz (6), der dazu angepasst und konfiguriert ist, unlösbar mit dem Anschlussstück (2) verbunden oder gekoppelt zu sein und der dazu ausgelegt ist, an einem Anschlussstutzen (9) des externen Behälters (31) lösbar montiert zu werden, um den externen Behälter (31) fluidisch mit der Blutbehandlungsmaschine (21) zu verbinden.

2. Anschlusseinheit (1) gemäß Anspruch 1, **dadurch gekennzeichnet, dass** der Aufsatz (6) als Codiereinheit vorgesehen ist, derart, dass er infolge individualer Dimensionierung oder Formgebung ausschließlich auf dem Anschlussstutzen (9) eines externen Behälters (31) eines dem Inhalt des Behälters (31) entsprechenden, vorherbestimmten Typs montierbar ist, wodurch sichergestellt ist, dass ausschließlich der externe Behälter (31) des vorherbestimmten, dem Inhalt des Behälters entsprechenden Typs mittels der Anschlusseinheit (1) fluidisch an der Blutbehandlungsmaschine (21) anschließbar ist.

3. Anschlusseinheit (1) gemäß Anspruch 1 oder 2, **dadurch gekennzeichnet, dass** der Aufsatz (6) dazu ausgelegt ist, durch Formschluss oder Reibschluss an dem Anschlussstutzen (9) des externen Behälters (31) eines dem Inhalt des Behälters (31) entsprechenden vorherbestimmten Typs gehalten zu werden, wenn der Aufsatz (6) auf dem Anschlussstutzen (9) montiert ist.

4. Anschlusseinheit (1) gemäß einem der vorhergehenden Ansprüche, **dadurch gekennzeichnet, dass** der Aufsatz (6) als ein formelastischer Balg ausgestaltet ist, welcher über den Anschlussstutzen (9) des externen Behälters (31) stülpbar ist.

5. Anschlusseinheit (1) gemäß einem der vorhergehenden Ansprüche 1 bis 3, **dadurch gekennzeichnet, dass** der Aufsatz (6) um die behälterseitige Leitung (4) drehbar und als formstabile Kappe ausgestaltet ist, welche vorzugsweise ein Innengewinde aufweist, welches dazu ausgelegt ist, mit einem entsprechenden Außengewinde des Anschlussstutzens (9) des externen Behälters (31) des vorherbestimmten Typs in Eingriff gebracht zu werden.

6. Anschlusseinheit (1) gemäß einem der vorhergehenden Ansprüche, weiterhin **gekennzeichnet durch** zumindest einen behälterseitigen Anschlussnippel (8a), an welchem die behälterseitige Leitung 4 montierbar ist und der in seinem proximalen Längsabschnitt eine Verstärkung aufweist, welche sich im Bereich des Aufsatzes (6) und über den Bereich des Aufsatzes (6) in Richtung distal hinaus einen definierten Längenabschnitt entlang der behälterseitigen Leitung (4) erstreckt.

7. Anschlusseinheit (1) gemäß Anspruch 6, **dadurch gekennzeichnet, dass** im Bereich der Verstärkung sowie distal benachbart zu dem Aufsatz (6) ein Begrenzungselement (7) am Anschlussnippel (8a) vorgesehen ist, welches eine axiale Bewegung des Aufsatzes (6) entlang der Längsachse des Anschlussnippels (8a) begrenzt.

8. Anschlusseinheit (1) gemäß einem der Ansprüche 6 oder 7, weiterhin **gekennzeichnet durch** mindestens ein vorzugsweise zwei Dichtelement(e) (5a,5b), welche(s) an dem Anschlussnippel (8a) vorzugsweise im Bereich der Verstärkung angeordnet sind/ist und welche(s) dazu ausgelegt sind/ist, einen Zwischenraum zwischen der Außenseite des Anschlussnippels (8a) vorzugsweise der Verstärkung und einer Innenseite einer Aufnahme der Blutbehandlungsmaschine (21) und/oder des Anschlussstutzens (9) des externen Behälters fluidisch abzudichten, wenn das Verstärkungselement (8) in den Anschluss der Blutbehandlungsmaschine (21) oder den Anschlussstutzen des Behälters eingeführt ist.

9. Anschlusseinheit (1) gemäß einem der vorhergehenden Ansprüche, weiterhin **gekennzeichnet durch** eine Freigabevorrichtung (10), welche einen Fluidfluss zwischen der Anschlusseinheit (1) und dem externen Behälter (31) nur dann freigibt, wenn der Aufsatz (6) der Anschlusseinheit (1) in einer vorherbestimmten Montageposition an dem Anschlussstutzen (9) des externen Behälters (31) montiert ist.

10. Anschlusseinheit (1) nach Anspruch 9, **dadurch gekennzeichnet, dass** die Freigabevorrichtung (10) ein Reedkontakt, ein kapazitiver Sensor oder eine Lichtschranke ist.

11. System aus einer Anschlusseinheit (1) nach einem der Ansprüche 1 bis 10 und einem externen Behälter (31).

12. Blutbehandlungsmaschine (21) mit mindestens einer Anschlusseinheit (1) nach einem der Ansprüche 1 bis 10, welche über die maschinenseitige Leitung (3) der Anschlusseinheit (1) mit einem vorherbestimmten Anschluss der Blutbehandlungsmaschine (21) fluidisch verbunden ist.

13. Blutbehandlungsmaschine (21) mit einer Mehrzahl an Anschlusseinheiten (1) nach einem der Ansprüche 1 bis 10, welche jeweils über ihre maschinenseitige Leitung (3) mit einem jeweiligen vorherbestimmten Anschluss der Blutbehandlungsmaschine (21) fluidisch verbunden sind, **dadurch gekennzeichnet, dass** der jeweilige Aufsatz (6) einer jeden Anschlusseinheit (1) der Mehrzahl an Anschlusseinheiten (1) derart ausgestaltet ist, dass er nur an dem Anschlussstutzen (9) eines jeweiligen zugehörigen vorherbestimmten Typs von externem Behälter (31), wobei der Typ des Behälters (31) dem Inhalt des Behälters (31) entspricht, lösbar montierbar ist.

14. Blutbehandlungsmaschine (21) nach einem der Ansprüche 10 oder 11, **dadurch gekennzeichnet, dass** jede der Anschlusseinheiten (1) der Blutbehandlungsmaschine (21) eine Freigabevorrichtung (10) aufweist, welche einen Fluidfluss zwischen der Blutbehandlungsmaschine (21) und einem jeweiligen externen Behälter (31) nur dann freigibt, wenn der Aufsatz (6) der Anschlusseinheit (1) korrekt in einer vorherbestimmten Montageposition an dem Anschlussstutzen (9) des jeweiligen externen Behälters (31) montiert ist.
